(19) Europäisches Patentamt
European Patent Office
Office européen des brevets

(11) **EP 2 708 596 B1**

(12) **EUROPEAN PATENT SPECIFICATION**

(45) Date of publication and mention of the grant of the patent:
**09.11.2016 Bulletin 2016/45**

(51) Int Cl.:
***C12N 5/04*** *(2006.01)* ***A61K 36/23*** *(2006.01)*

(21) Application number: **12382351.0**

(22) Date of filing: **14.09.2012**

(54) **Centella genus plant cell cultures, method of production and uses thereof**

Zellkulturen der Centella-genus-Pflanze, Verfahren zur Herstellung und Verwendung davon

Cultures de cellules de plantes genre centella, procédé de production et leurs utilisations

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**19.03.2014 Bulletin 2014/12**

(73) Proprietor: **Casen Recordati, S.L.**
**50180 Utebo (Zaragoza) (ES)**

(72) Inventors:
- **Tabuenca Navarro, Daniel**
  **50180 Utebo (ES)**
- **Bello Navarro, Marta**
  **50180 Utebo (ES)**
- **Jané Font, Albert**
  **08221 Terrassa (ES)**
- **Expósito Tarrés, Oscar**
  **08221 Terrassa (ES)**
- **Allué Creus, José**
  **08230 Matadepera (ES)**

(74) Representative: **ZBM Patents - Zea, Barlocci & Markvardsen**
**Plaza Catalunya, 1**
**08002 Barcelona (ES)**

(56) References cited:
- **MANGAS ET AL: "Producción de saponinas triterpénicas en cultivos in vitro de Centella asiatica", INTERNET CITATION, 1 January 2009 (2009-01-01), pages 1-13,i, XP002687700, Retrieved from the Internet: URL:http://tdx.cesca.cat/handle/10803/2622 [retrieved on 2012-11-16]**
- **MERCEDES BONFILL ET AL: "Production of centellosides and phytosterols in cell suspension cultures of", PLANT CELL, TISSUE AND ORGAN CULTURE, KLUWER ACADEMIC PUBLISHERS, DO, vol. 104, no. 1, 9 August 2010 (2010-08-09), pages 61-67, XP019866351, ISSN: 1573-5044, DOI: 10.1007/S11240-010-9804-7**
- **ZHEN-YU WANG ET AL: "Repeated elicitation enhances taxane production in suspension cultures of Taxus chinensis in bioreactors", BIOTECHNOLOGY LETTERS, vol. 24, no. 6, 1 January 2002 (2002-01-01), pages 445-448, XP055049041, ISSN: 0141-5492, DOI: 10.1023/A:1014549008516**
- **WEI WANG ET AL: "Enhancement of ginsenoside biosynthesis in high-density cultivation of Panax notoginseng cells by various strategies of methyl jasmonate elicitation", APPLIED MICROBIOLOGY AND BIOTECHNOLOGY, SPRINGER, BERLIN, DE, vol. 67, no. 6, 1 June 2005 (2005-06-01), pages 752-758, XP019331865, ISSN: 1432-0614, DOI: 10.1007/S00253-004-1831-Z**
- **CHENG X Y ET AL: "Improvement of phenylethanoid glycosides biosynthesis in Cistanche deserticola cell suspension cultures by chitosan elicitor", JOURNAL OF BIOTECHNOLOGY, ELSEVIER SCIENCE PUBLISHERS, AMSTERDAM, NL, vol. 121, no. 2, 24 January 2006 (2006-01-24), pages 253-260, XP024956918, ISSN: 0168-1656, DOI: 10.1016/J.JBIOTEC.2005.07.012 [retrieved on 2006-01-24]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ELLEN LAMBERT ET AL: "Modulation of Triterpene Saponin Production: In Vitro Cultures, Elicitation, and Metabolic Engineering", APPLIED BIOCHEMISTRY AND BIOTECHNOLOGY ; PART A: ENZYME ENGINEERING AND BIOTECHNOLOGY, HUMANA PRESS INC, NEW YORK, vol. 164, no. 2, 26 January 2011 (2011-01-26), pages 220-237, XP019891080, ISSN: 1559-0291, DOI: 10.1007/S12010-010-9129-3
- JACINDA T JAMES ET AL: "Characterisation of two phenotypes of Centella asiatica in Southern Africa through the composition of four triterpenoids in callus, cell suspensions and leaves", PLANT CELL, TISSUE AND ORGAN CULTURE, KLUWER ACADEMIC PUBLISHERS, DO, vol. 94, no. 1, 16 May 2008 (2008-05-16), pages 91-99, XP019615859, ISSN: 1573-5044
- JAMES JACINDA T ET AL: "Pentacyclic triterpenoids from the medicinal herb, Centella asiatica (L.) Urban.", MOLECULES (BASEL, SWITZERLAND) 2009, vol. 14, no. 10, 2009, pages 3922-3941, XP002691813, ISSN: 1420-3049


Remarks:

The file contains technical information submitted after the application was filed and not included in this specification

## Description

The present invention relates to plant cell cultures derived from a plant of the *Centella* genus. It also relates to methods for obtaining cultured cells enriched in secondary metabolites of interest, and to uses of these plant cell cultures in the field of medicine, health and welfare.

BACKGROUND ART

There exist several compositions including extracts from plants of the *Centella* genus, namely from *Centella asiatica* specie (*C. asiatica*), also named Gotu Kola, Tiger grass and Indian pennywort. The extracts of this plant have been widely used in the management of dermatological conditions, to treat wounds, scratches, superficial burns, eczemas, or as antiinflammatory agent.

The extended use of *Centella asiatica* extracts in regenerating skin compositions, as well as in damaged tissue repairing preparations, is due to the capability of some of the compounds in the plant, which are able to promote collagen synthesis. Some of these active constituents of *Centella asiatica* are pentacyclic triterpenoids, found as genins (asiatic and madecassic acid) and heterosides (asiaticoside and madecassoside) in the plant. Since these compounds are associated with *Centella,* they are also known as centellosides. Maquart et al., "Stimulation of collagen synthesis in fibroblast cultures by a triterpene extracted from Centella asiatica". Conn. Tissue Res -1990, vol. 24, pp. 107-120, disclose indeed that asiaticoside, asiatic and madecassic acid promote collagen synthesis in a dose-dependant manner.

Centellosides are a type of secondary metabolites present in *Centella asiatica,* and they are considered to be involved in the response to stress stimuli. Secondary metabolites are compounds synthesized by plant cells, and although not having an apparent function in the plant's primary metabolism, they play important secondary roles, for example by acting as insect repellants, defense molecules against microorganisms, or against external stimuli (cut of some parts of the plant, presence of poisons, etc.). Some of these plant-derived compounds are useful as active pharmacological ingredients. Nonetheless, the chemical synthesis of the same is sometimes difficult and for this reason they are extracted from the plants, and further concentrated and purified if required.

Examples of extracts of *Centella asiatica* include aqueous extracts, alcoholic extracts, and extracts with other solvents. Extraction is usually performed from different parts of the plant including leaves, stems, fruits, seeds, branches, etc.

Titrated Extract of *Centella asiatica* (TECA) is one of the mostly known extracts of this plant. It includes asiaticoside (40 % by weight), and asiatic and madecassic acid (60 % by weight). Madecassoside is not present, since due to the extraction process and due to the high solubility in water, it is eliminated during manipulation. The TECA is thus a standardized extract of *Centella asiatica,* in which the active ingredients are combined in constant proportions to guarantee optimal activity.

On the other hand, the patent document EP867447, filed by Dong Kook Pharmaceutical discloses an hydrosoluble extract including asiaticoside and madecassoside, wherein said asiaticoside and madecassoside are in a ratio ranging from 4:6 to 6:4, both components constituting at least 97 % by weight or more of the extract.

Extraction processes, though standardized, carry out the problem that become quite expensive, especially when the aim is a composition enriched in one specific compound or group of compounds of interest from the selected plant.

An alternative way to obtain a compound of interest from a plant acting as factory is by using the technology of plant cell cultures. Plant cell cultures allow the isolation of cells from different parts of the plant (leaves, fruits, stem, roots, shoots, etc.), which parts of the plant are further cultured under controlled conditions in order to get a desired compound. With the technology of plant cell cultures, isolated plant cells are generally submitted to cell undifferentiation until totipotency is reached. These undifferentiated totipotent cells can then become any plant cell tissue upon adequate stimulation. It is for this reason that these undifferentiated cells are also named plant stem cells.

These totipotent plant cells are used as factories of metabolites or compounds of interest once they are stimulated with specific compounds (also known as eliciting agents). This technology is exemplified in the document of Mulabagal et al., "Plant cell cultures-an alternative and efficient source for the production of biologically important secondary metabolites", International Journal of Applied Science, Engineering and Technology-2004, Vol. 2, pp.: 101-153. Mulabagal et al. disclose callus and suspension culture methods for the production of bioactive secondary metabolites from medicinal plants.

The principle advantage of the technology of plant cell cultures is that it allows the provision of a continuous and reliable source of plant-derived pharmaceuticals.

In the particular case of *Centella asiatica,* a suspension of undifferentiated totipotent cells have been used to obtain phytosterols and centellosides. The procedure is disclosed in Bonfill et al., "Production of centellosides and phytosterols in cell suspension cultures of Centella asiatica", Plant Cell Tiss Organ Cult - 2011, Vol. No. 104, pp.: 61-67. In this document *C. asiatica* cell suspensions were established and treated with two concentrations (100 and 200 μM)

of the eliciting agent (stimulator) methyl jasmonate (MeJA). The maximum centelloside production was observed in the stationary growth phase, reaching 0.16 mg/g of dry weight at day 25 of the culture in the control and 1.11 mg/g at day 15 in the MeJA-elicited cultures. By means of this methodology a percentage by weight of 54.5 % of madecassoside and of 45.5 % of asiaticoside in respect of the total amount of centellosides was obtained.

Although all known compositions comprising components of *Centella asiatica* (mainly extracts of the plant) have interesting properties in terms of promoting tissue repair, especially dermatological tissue, there is still a need of alternative compositions with high capability of inducing collagen synthesis.

SUMMARY OF THE INVENTION

It is herewith provided a cell culture of a plant of *Centella* genus, which is specifically enriched in madecassoside and has good properties as collagen synthesis inducing agent.

Thus, in a first aspect the invention relates to a plant cell culture from a plant of the *Centella* genus, said plant cell culture comprising an amount of madecassoside from 85 % to 95 % by weight, and an amount of asiaticoside from 5 % to 15 % by weight, being % by weight both of madecassoside and asiaticoside in respect of the total weight of centellosides in the cell culture, and obtainable by:

a) cultivating by cell suspension an undifferentiated plant cell from a plant of the *Centella* genus in a suitable growing medium for a period comprised from 8 to 15 days in which stationary growing phase is reached, to obtain an in-stationary phase culture;

b) adding 3 doses of a jasmonate compound to the in-stationary phase culture of step a), wherein each one of the doses added corresponds to an amount such of jasmonate compound that the final concentration at the end of each addition is from 80 μM to 150 μM; and wherein

the three doses of the jasmonate compound are added with the following schedule:

(i) the first dose once the culture of step a) has reached the stationary growing phase;
(ii) the second dose is added from 3 to 5 days later in respect of the first addition;
(iii) the third dose is added from 8 to 11 days later in respect of the first addition.

Major centellosides are, as above indicated, the genins asiatic and madecassic acid, and also the heterosides asiaticoside and madecassoside, the four represented in formulas (I) to (IV) below:

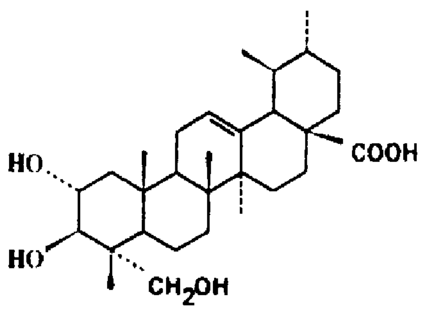

(I) Asiatic acid

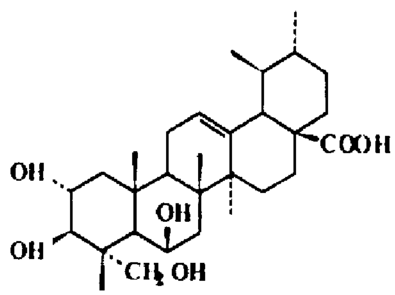

(II) Madecassic acid

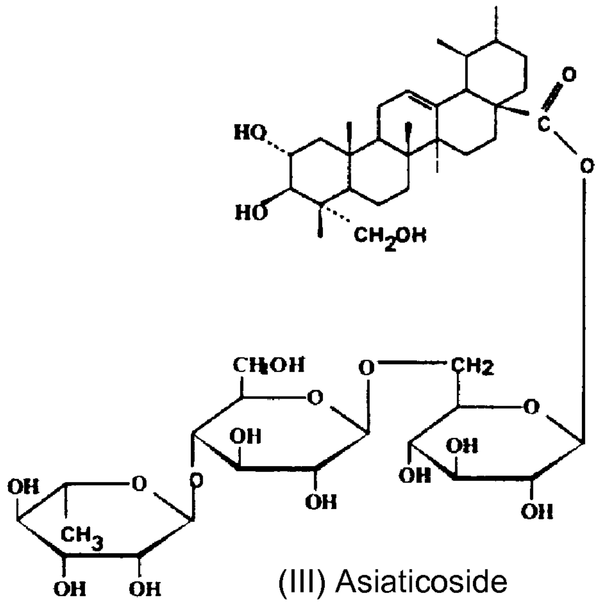

(III) Asiaticoside

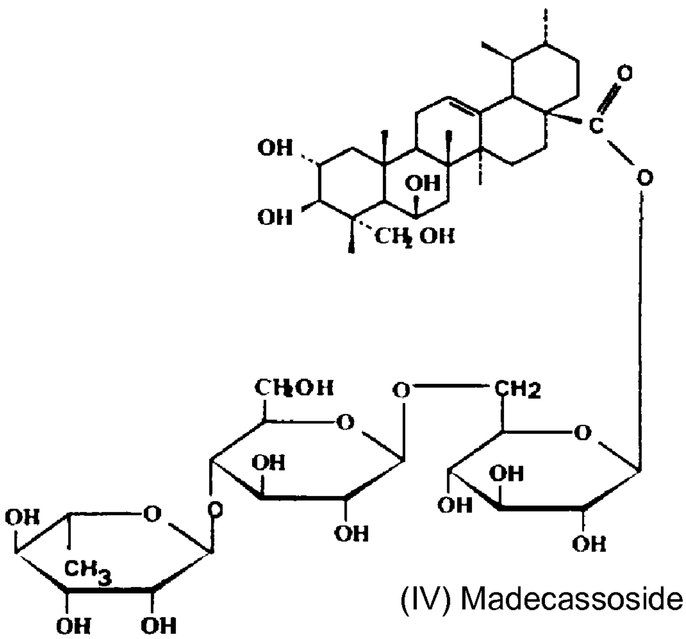

(IV) Madecassoside

Surprisingly, plant cell culture from a plant of the *Centella* genus has a concrete profile of centelloside concentrations. Particularly, the cell culture is enriched in madecassoside comprising an amount of madecassoside from 85 % to 95 % by weight, and an amount of asiaticoside from 5 % to 15 % by weight. In addition, these plant cell cultures of the invention efficiently promote the synthesis of collagen, which in turn allows the healing and repair of damaged dermatological tissue, being thus usable as tissue repair agents.

Use of the plant cell cultures technology, and especially suspension cultures, allow obtaining the products of interest in a highly reproducible way, with manufacture batches being as similar as possible. This is a great advantage of the plant cell cultures technology, because reproducibility is an obliged condition for agents to be approved by regulatory authorities and to be used in pharmaceutical compositions. Additionally, the cell suspension cultures are of particular interest since they are scalable and lead, moreover, to high process yields with a lower cost than the extraction processes applied to the plants.

A further aspect of the invention is a plant cell culture as defined above for use as a medicament.

BRIEF DESCRIPTION OF THE DRAWINGS

FIG. 1, related to Example 3, is a bar diagram showing the amount of collagen ($\mu g/10^6$ human fibroblast cells) synthesized by human fibroblasts after the addition, in the culture medium, of different compositions including the plant cell culture of the *Centella* genus according to the invention.

FIG. 2, related to Example 1, is another graphic with bars showing the amount (concentration in mg/L) of madecassoside (M) and of asiaticoside (A) obtained in a cell suspension of a plant of the *Centella* genus submitted to the method according to the invention. T means treatment or addition of methyl jasmonate; 1X means one addition of methyl jasmonate; 3X means three addition of methyl jasmonate.

DETAILED DESCRIPTION OF THE INVENTION

The following definitions are included for the purpose of understanding.

The term "enriched" means herewith that madecassoside is the major component of the total of centellosides produced by the *Centella* genus plant cell culture, namely representing at least 85 % by weight of the total of centellosides (madecassoside, asiaticoside, madecassic acid and asiatic acid) in the plant cell culture. The amount in percentage by weight of madecassoside in the cell culture is, in fact, the representation of the amount in any individual cell constituting said culture, any cell contributing to the final absolute amount of the compound.

The amount of centellosides is preferably determined in the culture following the HPLC methodology. Cells are lyophilized and extracted through methanol:water extraction, and then dissolved in a polar solvent to perform the HPLC analysis.

In the sense of the present invention a "plant cell culture" are plant cells derived from tissue or cells of plants, which are then cultured in a container or recipient. The plant cell cultures include callus tissues (callus), differentiated cultured tissues or a cultured organ tissue. The callus are, in turn, cultured plant cells aggregates consisting of undifferentiated cells (mass of undifferentiated plant cells) propagated over solid culture medium containing plant hormones or in liquid culture medium containing the plant hormones. On the other hand, the differentiated cultured tissues are aggregates of plant cells constituting a root, a stem, a bud or a fruit.

Plant "callus" is a mass of unorganized parenchymatic cells derived from plant tissue (explants) for use in biological research and biotechnology. In plant biology, callus cells are those cells that cover a plant wound. Callus formation is induced from plant tissues after surface sterilization and plating onto *in vitro* tissue culture medium. Plant growth regulators, such as auxins, cytokinins, and gibberellins, are supplemented into the medium to initiate callus formation or somatic embryogenesis.

A "plant cell culture lysate" is a plant cell culture which has been submitted to disaggregation and disruption of the cells, by means of sonication or mechanically, via a cutting-agitator. With the disruption of the cells the content of the cytoplasm is delivered in the medium (solid or liquid), thus leading to a mixture that comprises the different components of the cell (compounds of the cytoplasm, membrane fragments and compounds from the cell previously delivered in the medium). The plant cell culture lysate can also be obtained by other means including, as a non-limitative example, freezing at very low temperature.

In the sense of the invention a "cell suspension culture" or "cell suspension" is a group of undifferentiated cells dispersed or in suspension in a liquid nutrient medium (liquid culture medium). The cell suspension may comprise cells in various stages of aggregation.

In the sense of the invention an "in-stationary phase culture" or "growing culture", used herewith interchangeable, means a culture of cells (undifferentiated or differentiated) from a plant of the *Centella* genus that is grown until a desired phase, in which phase the density of the cell culture is maintained at a constant value because cells neither die nor grow. To this culture further treatments can be applied.

The term "jasmonate derivative compound" or "jasmonate compound", used herewith interchangeable, encompasses compounds derived from jasmonic acid of formula (A), in which one or several hydrogen atoms, namely the hydrogen atom of the hydroxyl compound, have been substituted by a ($C_1$-$C_3$)-alkyl radical. Examples of ($C_1$-$C_3$)-alkyl radical include methyl, ethyl, and propyl. Thus, some of the jasmonate derivatives compounds are methyl jasmonate (formula (B)), ethyl jasmonate and propyl jasmonate.

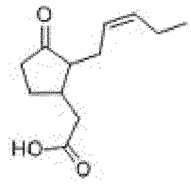

(A)

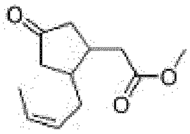

(B)

A "tissue repair agent" is to be understood as a compound or mixture of compounds (in this invention a plant cell culture or a plant cell culture lysate) which due to its inherent capability of recovering damaged cells, or interstitial cell structures, namely skin or mucosa cells, promote the recovering of the entire functionality of the tissue. An example of tissue repair agent is a compound or mixture of compounds that allows the synthesis of collagen.

The term "pharmaceutically acceptable" as used herein pertains to compounds, materials, compositions, and/or dosage forms which are, within the scope of sound medical judgment, suitable for use in contact with the tissues of a subject (e.g. human) without excessive toxicity, irritation, allergic response, or other problem or complication, commensurate with a reasonable benefit/risk ratio. Each carrier, excipient, etc., must also be "acceptable" in the sense of being compatible with the other ingredients of the formulation. Suitable carriers, excipients, etc. can be found in standard pharmaceutical texts, and include, as a way of example preservatives, agglutinants, humectants, emollients, and antioxidants.

The term "effective amount" as used herein, means an amount of an active agent high enough to deliver the desired benefit (either the treatment or prevention of the illness), but low enough to avoid serious side effects within the scope of medical judgment.

The term "cosmetically acceptable" or "dermatological acceptable", which is herein used interchangeably, refers to the excipients or carriers suitable for use in contact with human skin without undue toxicity, incompatibility, instability, allergic response, among others.

The term "medical device" means as defined by the European Directive 2007/47/EC any product, instrument, device, apparatus, computer program, material or article used in the medico sanitary field and being regulated by the European Directive 90/385/CEE of active implantable medical device, 98/79/CE of medical devices for in vitro diagnostic, and 93/42/CEE for general medical devices. The medical devices are included in the sanitary technologies. They are used in human beings for the diagnostic, prevention, control, treatment or alleviation of a disease, for the compensation of a deficiency; for investigating, substituting or modification of anatomy or a physiologic process; and for the regulation of contraception. Medical devices of the present invention relate to compositions or formulations ("medical device compositions"), or to products or devices comprising said compositions or formulations, comprising an effective amount of a plant cell culture from a plant of the *Centella* genus, said plant cell culture comprising an amount of madecassoside from 85 % to 95 % by weight, and an amount of asiaticoside from 5 % to 15 % by weight, in respect of the total weight of centellosides in the cell culture, and obtainable by the method defined above. The term "medical device composition" is used herein to identify the compositions according to the invention, which compositions, as such (by their selves) are catalogued by the competent sanitary authorities as medical devices.

For the purposes of the invention, any ranges given include both the lower and the upper end-points of the range.

Most preferred amounts of madecassoside are 86 %, 87 %, 88 %, 89 %, 90 %, 91 %, 92 %, 93 %, 94 %, and 95 % by weight in respect of the total weight of centellosides. Specially preferred is an amount of 90 % by weight of madecassoside in respect of the total weight of centellosides in the cell culture.

In another preferred embodiment, the plant cell culture according to the invention comprises an amount of madecassoside from 85 % to 95 % by weight in respect of the total weight of centellosides in the plant cell culture; and an amount of asiaticoside which goes from 5 % to 15 % by weight in respect of the total weight of centellosides in the plant cell culture, thus representing both 100 % by weight of the centellosides of the cell culture. Most preferably, the plant cell culture of *Centella* genus comprises an amount of 90 % by weight of madecassoside in respect of the total weight of centellosides, and an amount of 10 % by weight of asiaticoside in respect of the total weight of centellosides.

In another embodiment, both madecassoside and asiaticoside represent the majoritary of the centellosides in the cell culture, both being at least the 97 %, at least the 98 %, at least the 99 %, or at least 99.9 %, by weight of the total weight of centellosides in the cell culture, and wherein the proportion of madecassoside is by weight of the total weight of centellosides in the cell culture, preferably 85 % and most preferably 90 %. Other centellosides which can be present are asiatic acid and madecassic acid, which in the cell culture according to the invention are generally present in minor amounts.

Also another preferred embodiment is a plant cell culture wherein the plant of the *Centella* genus is *Centella asiatica.* The plant cell culture comes from an undifferentiated cell. *Centella asiatica* can be of different territorial origin.

Examples of said origins include Asiatic countries, such as China, India, Indonesia, Pakistan, Sri Lanka, African countries, such as Madagascar, oriental European countries, and American countries, such as Mexico.

In another embodiment, the plant cell culture is an undifferentiated plant cell culture of *Centella asiatica* wherein said undifferentiated plant cell comes from a source of well-stablished undifferentiated cells.

In another embodiment, the plant cell culture of the invention comes from a differentiated cell source (from any of the parts of the plant of the *Centella* genus), said differentiated cells being previously submitted to a treatment for the induction of calli (mass of undifferentiated plant cells) in order to produce the madecassoside amounts. Examples of calli induction media include the Murashige and Skoog (MS) supplemented with sugar (sucrose), 6-benzyladenine (BA) and 1-napthaleneacetic acid (NAA). Once induced the calli, examples of growing mediums include MS supplemented with sugars (sucrose), 2,4-dichlorophenoxyacetic acid (2,4-D) and of N1-(2-chloro-4-pyridyl)-N2 phenylurea (4PU-30).

Yet in another preferred embodiment, the plant cell culture according to the invention is in the form of a plant cell culture lysate, also named lysate plant cell culture, or simply, lysate.

The plant cell culture lysate is preferably obtained by mechanical disruption of the cells of the plant cell culture, and further filtration through a mesh from 50 to 150 $\mu$m. Other means for lysing the plant cell cultures are also applicable provided that the final mixture is proper for being administered or used in derivative products, such as pharmaceutical compositions. Examples of such means include freezing at very low temperature.

The plant cell culture according to the invention is obtainable by a method that includes:

a) cultivating by cell suspension an undifferentiated plant cell from a plant cell from a plant of the *Centella* genus to obtain and in-stationary phase culture, and
b) adding 3 doses of a jasmonate compound to the in-stationary phase culture of step a), wherein each one of the doses added corresponds to an amount such of jasmonate compound that the final concentration at the end of each addition is from 80 $\mu$M to 150 $\mu$M; and wherein

the three doses of the jasmonate compound are added with the following schedule:

(i) the first dose once the culture of step a) has reached the stationary growing phase;
(ii) the second dose is added from 3 to 5 days later in respect of the first addition;
(iii) the third dose is added from 8 to 11 days later in respect of the first addition.

The first dose is generally the dose which starts step b) of the method of obtaining the plant cell culture. The step a) of growing or cultivating is generally controlled or followed by means of turbidimetric assays and packing volum determination.

This embodiment can also be formulated as a method of obtaining a plant cell culture as defined above, wherein the jasmonate compound is added three times with the following schedule: at the beginning of step b), that is, when the growing culture has reached its stationary growing-phase; from 3 to 5 days later in respect of the first addition; and from 8 to 11 days later in respect of the first addition.

In a preferred embodiment of the plant cell culture obtainable by the method according to the invention, the undifferentiated plant cells of step a) are cells from a subcultured callus source. This callus source can be obtained from explants of different parts of a plant of *Centella* genus, which explants are treated in a suitable medium to finally obtain an established solidified callus culture. Examples of media for obtaining calli have been disclosed above and include, among others, MS medium supplemented with sugar and other compounds. Alternatively, the undifferentiated plant cells of step a) are cells from other undifferentiated cell suspension cultures.

Suitable mediums for the cell suspension culture include Murashige & Skoog (MS) medium, Gamborg's (B5) and Linsmaier & Skoog (LS).

In another preferred embodiment, the cells of step a) are derived from fruit, leaves, shoots, buds, roots, stems, seeds and flowers. Alternatively, the cells of step a) are derived from the meristemic tissue. The meristem is the tissue in most plants consisting of undifferentiated cells (meristematic cells), found in zones of the plant where growth can take place. The meristematic cells give rise to various organs of the plant, and keep the plant growing. The meristematic cells are frequently compared to the stem cells in animals, that have an analogous behavior and function, since they do not possess any defined end fate.

In a preferred embodiment of the plant cell culture obtainable by the method of the invention, the jasmonate compound is provided within the suspension culture at preferred final concentrations of jasmonate compound from 90 $\mu$M to 120 $\mu$M. Most preferred from 95 $\mu$M to 100 $\mu$M. Also preferred are concentrations of 96, 97, 98, 99, and 100 $\mu$M.

The jasmonate compound is preferably selected in such a way that it is a soluble-in-water compound. This allows it to be easily applied in the liquid medium culture. Nonetheless, also jasmonic acid can be used. In the same way if solubility is forced with appropiate conditions of pressure those volatile jasmonate compounds can also be used. Alternatively, volatile compounds can be used if the method is performed in a saturated atmosphere of such desired

jasmonate compound, in order to force liquid/gas equilibrium for achieving the desired concentration in the culture.

In a preferred embodiment, the jasmonate compound is selected from the group consisting of methyl jasmonate, ethyl jasmonate, and propyl jasmonate being highly preferred the methyl jasmonate applied in the schedules and concentrations as above indicated.

The plant cell culture of the invention is obtainable by a method that further comprises the step of lysing the plant cell culture once the period or step including the eliciting schedule (step b, with three doses of a jasmonate derivative compound) has been finalized.

In a preferred embodiment, the lysis is performed with an homogenizing device and then the homogenized mixture is filtered through a filter with a size pore diameter comprised from from 50 to 150 $\mu$m, preferably a filter of 100 $\mu$m of pore diameter. The filtered cell culture lysate can then be submitted to lyophilization or spray drying in order to evaporate water (mainly) and for preserving the lysate. Alternatively, the cell culture lysate can be kept under controlled temperature in liquid suspension and if needed appropriate conservatives are added.

As will be illustrated by way of examples, the plant cell culture according to the invention, as well as the plant cell culture lysate, can be used as a medicament.

Thus, the plant cell culture of the invention with the percentage by weight of madecassoside comprised from 85 % to 95 % by weight, and an amount of asiaticoside from 5 % to 15 % in respect of the total weight of centellosides can be applied directly or as an ingredient of a pharmaceutical composition. The plant cells can be added as entire cells, as well as in the form of lysate cell culture. The pharmaceutical composition comprises a therapeutically effective amount of the plant cell culture of the invention together with any acceptable pharmaceutical excipient and/or carrier.

In an embodiment, the plant cell culture it is preferably for use as tissue repair agent. This aspect can also be formulated as a method for treating and/or preventing tissue damage, namely topical skin (epidermis) or mucosa tissue damage, the method comprising administering a plant cell culture of the *Centella* genus, comprising an amount of madecassoside from 85 % to 95 % by weight, and an amount of asiaticoside from 5 % to 15 % by weight, being % by weight both of madecassoside and asiaticoside in respect of the total weight of centellosides in the cell culture, and obtainable by the method disclosed above,to the subject in need thereof. This aspect can alternatively be formulated as the use of a plant cell culture of the *Centella* genus, comprising an amount of madecassoside from 85 % to 95 % by weight, and an amount of asiaticoside from 5 % to 15 % by weight, being % by weight both of madecassoside and asiaticoside in respect of the total weight of centellosides in the cell culture, and obtainable by the method disclosed above, for the manufacture of a medicament for the treatment and/ or prevention of tissue damage, namely topical skin (epidermis) or mucosa tissue damage.

The plant cell culture according to the invention, as well as the plant cell culture lysate, can also be used as a cosmetic agent. Thus, the plant cell culture of the invention with the percentage by weight of madecassoside comprised from 85 % to 95 % and an amount of asiaticoside from 5 % to 15 % by weight, in respect of the total weight of centellosides, can be applied directly or as an ingredient of a cosmetic composition. The plant cells can be added as entire cells, as well as in the form of lysate cell culture. The cosmetic composition comprises a cosmetically effective amount of the plant cell culture of the invention together with any acceptable cosmetic excipient and/or carrier.

Additionally, the plant cell culture according to the invention, as well as the plant cell culture lysate, can also be used as a medical device once formulated as ingredients in medical device compositions. Thus, the plant cell culture of the invention with the percentage by weight of madecassoside comprised from 85 % to 95 % and an amount of asiaticoside from 5 % to 15 % by weight, in respect of the total weight of centellosides can be applied directly or as an ingredient of a medical device. The plant cells can be added as entire cells, as well as in the form of lysate cell culture. The medical device composition comprises an effective amount of the plant cell culture of the invention together with any acceptable excipient and/or carrier and/or any other components defining the medical device (i.e.: any support, or woven or non-woven fabric).

Throughout the description and claims the word "comprise" and variations of the word, are not intended to exclude other technical features, additives, components, or steps. Furthermore, the word "comprise" encompasses the case of "consisting of". The following examples and drawings are provided by way of illustration, and they are not intended to be limiting of the present invention. Furthermore, the present invention covers all possible combinations of particular and preferred embodiments described herein.

EXAMPLES

<u>Example 1:</u> Obtention of plant cell cultures of *Centella asiatica.*

Calli were obtained using Murashige and Skoog (MS) medium supplemented with 25-40 g $l^{-1}$ of sucrose (for example, 35 g $l^{-1}$), between 2-4 mg $l^{-1}$ of 6-benzyladenine (BA) (for example 3.3 mg $l^{-1}$) and between 2-4 mg $l^{-1}$ of 1-naphthaleneacetic acid (NAA) (for example, 2.5 mg $l^{-1}$), which provided good callus induction. As a growth medium, MS medium was used supplemented with 25-40 g $l^{-1}$ of sucrose (for example 40 g $l^{-1}$), between 1.5-4 mg $l^{-1}$ of 2,4-dichlorophenoxyacetic acid (2,4-D) (for example, 2.5 mg $l^{-1}$) and between 2.5-4 mg $l^{-1}$ of N1-(2-chloro-4-pyridyl)-N2 phenylurea

(4PU-30) (for example (2.5 mg $l^{-1}$) to obtain large and friable calli.

To establish cell suspension cultures, callus pieces (20 g) were inoculated into 500-ml flasks containing 200 ml of MS liquid medium supplemented with 20-30 g of sucrose (for example, 20 g), between 1-3 mg $l^{-1}$ of 2,4-D (for example, 1 mg $l^{-1}$) and between 0.1-1 g $l^{-1}$ of BA (for example, 0.8 g $l^{-1}$) and placed in a rotary shaker at 100 rpm in the dark at 25 °C. Cell cultures were passed through a 60-μm nylon filter every 2 weeks for 6 months to obtain a fine cell suspension culture.

For the cell suspension assays, 20 g of cells (fresh weight, FW) were inoculated into 200 ml of the aforementioned liquid medium in 500-ml flasks, with or without the elicitor, and were maintained in a rotary shaker at 100 rpm in the dark at 25 °C. After 12 days of growing (when stationary growing phase was reached), the cell suspension was elicitated with 100 μM methyl jasmonate (MeJA) at days 12, 16 and 22 from the initiation of the suspension. Other possible elicitation days include, a part of the day in which stationary growing phase is reached and the elicitation schedule is initiated, the days comprised from 3 to 5 days later of the first elicitation; and the days comprised from 8 to 11 later of the first elicitation.

After the eliciting period, the elicited culture was lysed with an homogenizer at 10000-15000 rpm, and filtered through a nylon mesh with a diameter pore of 100 μm.

The mixture contained:

- compounds of the culture medium not consumed by the cells;
- compounds secreted by the cells to the medium;
- soluble intracellular compounds; and
- not soluble cell membrane compounds with a size lower than 100 μm.

The final product was a cell culture lysate with high contents of madecassoside in respect of the total centellosides in the *C. asiatica* cells.

HPLC analysis of the cell culture lysate indicated that the amount of madecassoside was 90 % by weight of the total weight of centellosides (asiaticoside, asiatic acid, and madecassic acid representing 10 % by weight of the total weight of centellosides).

The amounts of centellosides were determined as indicated in Example 2 below. The areas under the curves (or peaks, a.u.c) of each centelloside of the HPLC analysis were calculated and correlated with the amount of each compound. Finally, the percentage by weight of each centelloside (madecassoside, asiaticoside, madecassic acid, and asiatic acid) in relation to the total weight of centellosides in the lysate were determined as:

$$\% = [(\text{a.u.c of a specific centelloside}) / (\sum \text{a.u.c of all centellosides})] \times 100$$

In FIG. 2, it is shown the amount (mg/L) of madecassoside (M) and of asiaticoside (A) obtained in the cell suspension culture with methyl jasmonate (100 μM). Left bars of the figure show the amounts determined with one (1X) addition (or treatment; (T)) of methyl jasmonate. Right bars correspond to the amounts of madecassoside (M) and asiaticoside (A) obtained with the elicitation schedule disclosed above; that is, three additions (3X) at days 12, 16 and 22 from the initiation of the suspension, and determined at day 27 from the initiation of the suspension. Data depicted in FIG. 2 correspond to the total concentrations from the cell fraction and the culture liquid medium fraction.

As can be seen, with the three additions of methyl jasmonate the absolute amount of madecassoside was from 8 to 9 times higher than the amount of asiaticoside, namely 8,5 times higher.

<u>Example 2.</u> Determination of centellosides (HPLC).

The quantification of the four centellosides (asiaticoside, madecassoside, asiatic acid and madecassic acid) was carried out through high-performance liquid chromatography (HPLC).

For cells extraction,1 g of fine powder (lyophilised) was first sonicated in methanol:water (9:1, v/v; 2 x 25 mL) for 2 h at room temperature and filtered through a 0.45 μm filter (Waters Millipore). The filtrate was evaporated to dryness. The dried extract was disolved in 1 mL of methanol and filtered through a 0.45 μm filter (Waters Millipore) and the clear filtrate was used for HPLC analysis. HPLC-UV analysis was performed at room temperature with a Spherisorb 5μm ODS2 4.6x250 mm column (Waters, Milford, MA, USA) using gradient elution, the eluents being acetonitrile (A) and water with ammonium dihydrogen phosphate 10 mM (pH 2.5 with orthophosphoric acid) (B) according to the following profile: 0-15 min, 80% A; 15-30 min, 62% A; 30-37 min, 30% A; 37-40 min, 80% A. The flow rate was 1 ml min-1 and the detector was set at 214 nm.

Example 3. Comparative assay in an *in vitro* model

In order to illustrate the advantageous effects of the plant cell culture of the invention, following it is disclosed an assay with different compositions comprising a cell culture lysate of *Centella asiatica* according to the invention. The capability of the compositions of inducing collagen synthesis of this cell culture lysate is tested in a culture of human dermal fibroblasts.

Table 1 show the characteristics of the tested compositions.

Table 1. Tested products

| Raw material | Concentration (% by weight) | | | |
|---|---|---|---|---|
| | PR/52/A | PR/53/A | PR/54/A | PR/55/A |
| *Centella asiatica* cell culture lysate | 10,000 | 5,000 | 1,000 | 0 |
| *Centella asiatica* extract with a 90 % by weight of madecassoside in respect of all centellosides (from Guangxi Changzhou Natural products Development (China); powder extract of the dried whole plant) | 0 | 0 | 0 | 0,500 |
| Excipients (solvents, emollients, pH buffers, gelling agents, antioxidants and preservative agents) | qsp. 100 * | | | |
| * "q.s.p" means "as needed for". | | | | |

The aim of this assay was to see the effect of the formulations listed in Table 1. Three of them (PR/52/A; PR/53/A; and PR/54/A) contained a *Centella asiatica* cell culture lysate, prepared as exposed in Example 1. They contained an amount of madecassoside comprised from 85 % to 95 %, namely 90 % by weight in respect of the total weight of centellosides obtained after the treatment with the elicitor methyl jasmonate. The other formulation (PR/55/A) contained an amount of a *Centella asiatica* extract.

The test consisted in the evaluation of the effect in the collagen synthesis in human dermis fibroblasts after 24 hours of treatment with these formulations, each one applied at different non-toxic concentrations (100, 250 and 500 μg/ml) previously determined.

Collagen was determined with a colorimetric assay using the coloring agent Sirius Red (Direct red 80), which binds specifically to a triple helix sequence of the native collagen. As positive control (Control +) the beta transforming growing factor (TGF-β) was used. As negative control (Control -) culture medium was added.

Cells were cultivated at 37 °C for 24 hours (50 x$10^3$ cel/$cm^2$). The tested formulation was prepared and added to the cells at the three above indicated concentrations and incubated at 37 °C for 24 additional hours. For every tested concentration the following determinations were performed: a) collagen quantification, and b) cell quantification (Trypsin: 0,2 % w/v).

The results of the assay are depicted in FIG. 1, wherein the amount of collagen in terms of μg/$10^6$ human fibroblast cells is indicated in form of bars for every concentration (100, 250, and 500 μg/ml) tested for each formulation. When three bars are showed for a determined formulation, left bar shows the result at a final concentration in the culture of 100 μg/ml; the bar in the middle shows the results at 250 μg/ml; and right bar shows the results at 500 μg/ml. Vertical lines show the standard deviation of the results.

Cell growth was similar in negative control and in the cultures treated with the formulations, thus confirming that the assayed concentrations (or selected doses) of 100, 250, and 500 μg/ml were non-cytotoxic.

As can be deduced from this FIG. 1, the formulation containing 10 % by weight of the *Centella asiatica* cell culture lysate of the invention (PR/52/A) was able to stimulate collagen secretion in the culture medium from 100 to 500 μg/ml, being maximal at 250 μg/ml. This means 42 % more in respect of the negative control. The stimulation represented in turn the 80 % of the positive control stimulation (TGF-β).

On the other hand, the formulation containing 5 % by weight of the *Centella asiatica* cell culture lysate of the invention (PR/53/A) stimulated the collagen secretion (synthesis) between 100 and 500 μg/ml, also being maximal at 250 μg/ml. This represented 65 % more in respect of the negative control. The stimulation was noteworthy the 127 % in relation of the induction by the positive control.

The formulation containing 1 % by weight of the *Centella asiatica* cell culture lysate of the invention (PR/54/A) stimulated the collagen secretion (synthesis) between 100 and 250 μg/ml, also being maximal at 250 μg/ml. The amount of secreted collagen represented an increase of 31 % in respect of the negative control.

Finally, the formulation comprising 0,5 % of an extract of *Centella asiatica* with 90 % of madecassoside

(PR/55/A), that is, a powder of an extract of the dried whole plant, purified and titrated (TECA), stimulated the collagen secretion (synthesis) between 100 and 500 μg/ml, being maximal at 250 μg/ml. The amount of secreted collagen represented an increase of 55 % in respect of the negative control, and the increase was comparable with that of the positive control.

All these data allow concluding that the four formulations were able to stimulate the collagen synthesis, since the amount of collagen obtained in the cultured fibroblasts with all the concentrations of the four tested formulations obtained was greater than the amount in the negative control. Interestingly, the formulation comprising 5 % by weight of the *Centella asiatica* cell culture lysate of the invention (PR/53/A) had a similar effect, even greater, than the positive control. In addition, this formulation (5 % by weight of the *Centella asiatica* cell culture lysate of the invention) had an effect greater than the formulation comprising the extract of the plant.

Example 4. Composition with lysate from a plant cell culture of *Centella asiatica*

Next it is provided an example of qualitative composition including a *Centella asiatica* cell culture lysate which can be used as tissue repair agent.

This composition includes as active agent the lysate from a plant cell culture of *Centella asiatica* according to the invention, and as excipients solvents (i.e. water), viscosity agents, pH buffers, preservatives, antioxidants, and emolients. A non-limiting example is illustrared as follows:

| Ingredient |
|---|
| WATER |
| Lysate from a plant cell culture of *Centella asiatica* |
| Acrylates/C10-30 Alkyl Acrylate cross-polymer |
| TRIETANOLAMINE |
| IMIDAZOLIDINYL UREA |
| DISODIUM EDTATE |
| ASCORBIC ACID |
| PROPILENE GLYCOL |
| PROPYLPARABEN |
| METYLPARABEN |
| CAMOMILLE EXTRACT *(Chamomilla recutita* extract) |
| MALLOW EXTRACT (*malva sylvestris* extract) |

All the data exposed herewith confirm that the plant cell cultures from a plant of the *Centella* genus of the invention, are advantageous alternatives, even better options, in respect of the traditional extracts of the plant. First of all because the method to obtain the plant cell culture is highly reproducible and not as complex as an extraction process. Afterwards, because in case a great amount of one of the compounds produced by the plant *Centella asiatica* is desired, the plant cell culture of the invention and the method of obtaining it are easier than any process involving extraction in a suitable medium and further steps to enrich the extract in the desired compound. This is so because the invention provides an eliciting schedule with a specific eliciting agent that, in an unexpected way, gives raise to a selective enrichment in madecassoside with respect of the total centellosides produced during said eliciting schedule.

REFERENCES CITED IN THE APPLICATION

- Maquart et al., "Stimulation of collagen synthesis in fibroblast cultures by a triterpene extracted from Centella asiatica". Conn. Tissue Res-1990, vol. 24, pp. 107-120.
- EP867447.
- Mulabagal et al., "Plant cell cultures-an alternative and efficient source for the production of biologically important secondary metabolites", International Journal of Applied Science, Engineering and Technology-2004, Vol. 2, pp.:

101-153.
- Bonfill et al., "Production of centellosides and phytosterols in cell suspension cultures of Centella asiatica", Plant Cell Tiss Organ Cult-2011, Vol . No. 104, pp.: 61-67.

## Claims

1. A plant cell culture from a plant of the *Centella* genus, said plant cell culture comprising an amount of madecassoside from 85 % to 95 % by weight, and an amount of asiaticoside from 5 % to 15 % by weight, being % by weight both of madecassoside and asiaticoside in respect of the total weight of centellosides in the cell culture, and obtainable by:

   a) cultivating by cell suspension an undifferentiated plant cell from a plant of the *Centella* genus in a suitable growing medium for a period comprised from 8 to 15 days in which stationary growing phase is reached, to obtain an in-stationary phase culture;
   b) adding 3 doses of a jasmonate compound to the in-stationary phase culture of step a), wherein each one of the dosis added corresponds to an amount such of jasmonate compound that the final concentration at the end of each addition is from 80 $\mu$M to 150 $\mu$M; and wherein

   the three doses of the jasmonate compound are added with the following schedule:

   (i) the first dose once the culture of step a) has reached the stationary growing phase;
   (ii) the second dose is added from 3 to 5 days later in respect of the first addition;
   (iii) the third dose is added from 8 to 11 days later in respect of the first addition.

2. The plant cell culture according to claim 1 , wherein the madecassoside is in an amount of 90 % by weight of the total weight of centellosides in the cell culture.

3. The plant cell culture according to any one of claims 1 to 2, wherein the plant of the *Centella* genus is *Centella asiatica.*

4. The plant cell culture according to any one of claims 1 to 3, which is a lysate and is obtainable by lysing the plant cell culture after step (b).

5. The plant cell culture according to any one of claims 1 to 4, wherein the jasmonate compound is selected from the group consisting of methyl jasmonate, ethyl jasmonate and propyl jasmonate.

6. The plant cell culture according to any one of claims 1 to 5, wherein the jasmonate compound is methyl jasmonate.

7. A plant cell culture as defined in any one of claims 1 to 6, for use as a medicament.

8. The plant cell culture of claim 7 as tissue repair agent.

9. Pharmaceutical or cosmetic composition that comprises a therapeutically effective amount or a cosmetically effective amount of the plant cell culture as defined in any of claims 1-6, together with any acceptable pharmaceutical or cosmetic excipient and/or carriers.

## Patentansprüche

1. Eine Pflanzenzellkultur aus einer Pflanze der Gattung *Centella,* wobei die Pflanzenzellkultur einen Anteil an Madecassosid von 85 Gew.-% bis 95 Gew.-% und einen Anteil an Asiaticosid von 5 Gew.-% bis 15 Gew.-%, als Gewichtsprozent von Madecassosid und Asiaticosid bezogen auf das gesamte Gewicht von Centellosiden in der Zellkultur, umfasst, und erhältlich dadurch dass:

   a) eine undifferenzierte Pflanzenzelle aus einer Pflanze der Gattung *Centella* durch Zellsuspension in einem geeigneten Nährmedium für einen Zeitraum von 8 bis 15 Tagen, in dem stationäre Wachstumsphase erreicht wird, kultiviert wird, um eine Kultur in stationärer Phase zu erhalten;
   b) 3 Dosen von einer Jasmonatverbindung in die Kultur in stationärer Phase des Schritts a) hinzugefügt werden,

wobei jede hinzugefügte Dose einer Menge der Jasmonatverbindung entspricht, bei der die Endkonzentration am Ende von jeder Zugabe von 80 μM bis 150 μM reicht; und wobei

die drei Dosen der Jasmonatverbindung mit dem folgenden Zeitplan hinzugefügt werden:

(i) die erste Dose: wenn die Kultur des Schritts a) die stationäre Wachstumsphase erreicht hat;
(ii) die zweite Dose: von 3 bis 5 Tagen nach der ersten Zugabe;
(iii) die dritte Dose: von 8 bis 11 Tagen nach der ersten Zugabe.

**2.** Die Pflanzenzellkultur nach Anspruch 1, wobei das Madecassosid in einer Menge von 90 Gew.-% bezogen auf das gesamte Gewicht von Centellosiden in der Zellkultur vorhanden ist.

**3.** Die Pflanzenzellkultur nach einem der Ansprüche 1 bis 2, wobei die Pflanze der Gattung *Centella Centella asiatica* ist.

**4.** Die Pflanzenzellkultur nach einem der Ansprüche 1 bis 3, welche ein Lysat ist und durch Lyse der Pflanzenzellkultur nach dem Schritt (b) erhältlich ist.

**5.** Die Pflanzenzellkultur nach einem der Ansprüche 1 bis 4, wobei die Jasmonatverbindung ausgewählt aus der Gruppe bestehend aus Methyljasmonat, Ethyljasmonat und Propyljasmonat ist.

**6.** Die Pflanzenzellkultur nach einem der Ansprüche 1 bis 5, wobei die Jasmonatverbindung Methyljasmonat ist.

**7.** Eine Pflanzenzellkultur wie in einem der Ansprüche 1 bis 6 definiert, zur Verwendung als Arzneimittel.

**8.** Die Pflanzenzellkultur des Anspruchs 7 als Mittel zur Gewebereparatur.

**9.** Pharmazeutische oder kosmetische Zusammensetzung, die eine therapeutisch wirksame Menge oder eine kosmetisch wirksame Menge der Pflanzenzellkultur wie in einem der Ansprüche 1-6 definiert, zusammen mit akzeptablen pharmazeutischen oder kosmetischen Hilfsstoffen und/oder Trägerstoffen umfasst.

## Revendications

**1.** Une culture cellulaire végétale provenant d'une plante du genre *Centella,* comprenant ladite culture cellulaire végétale une quantité de madécassoside allant de 85 % à 95 % en poids, et une quantité d'asiaticoside allant de 5 % à 15 % en poids, étant le % en poids tant de madécassoside que d'asiaticoside par rapport au poids total de centellosides dans la culture cellulaire, et pouvant être obtenue par :

a) cultiver moyennant suspension cellulaire d'une cellule végétale indifférenciée d'une plante du genre *Centella* dans un milieu de culture approprié pendant une période de 8 à 15 jours dans laquelle ladite phase de croissance stationnaire est atteinte, afin d'obtenir une culture en phase stationnaire ;
b) additionner 3 doses d'un composé de jasmonate à la culture en phase stationnaire de l'étape a), où chacune des doses ajoutées correspond à une quantité du composé de jasmonate telle que la concentration finale à la fin de chaque addition va de 80 μM à 150 μM ; et dans laquelle

les trois doses du composé de jasmonate sont ajoutées avec le programme suivant :

(i) la première dose lorsque la culture de l'étape a) a atteint la phase stationnaire de croissance ;
(ii) la deuxième dose est ajoutée de 3 à 5 jours après la première addition ;
(iii) la troisième dose est ajoutée de 8 à 11 jours après la première addition.

**2.** La culture cellulaire végétale selon la revendication 1, dans laquelle le madécassoside est dans une quantité de 90 % en poids par rapport au poids total de centellosides dans la culture cellulaire.

**3.** La culture cellulaire végétale selon l'une quelconque des revendications 1 à 2, dans laquelle la plante du genre *Centella* est *Centella asiatica.*

**4.** La culture cellulaire végétale selon l'une quelconque des revendications 1 à 3, qui est un lysate qui peut être obtenu

en lysant la culture cellulaire végétale après l'étape (b).

5. La culture cellulaire végétale selon l'une quelconque des revendications 1 à 4, dans laquelle le composé de jasmonate est choisi dans le groupe qui consiste à jasmonate de méthyle, jasmonate d'éthyle et jasmonate de propyle.

6. La culture cellulaire végétale selon l'une quelconque des revendications 1 à 5, dans laquelle le composé de jasmonate est le jasmonate de méthyle.

7. Une culture cellulaire végétale telle que définie dans l'une quelconque des revendications 1 à 6, pour son utilisation en tant que médicament.

8. La culture cellulaire végétale de la revendication 7 en tant qu'agent de réparation tissulaire.

9. Composition pharmaceutique ou cosmétique qui comprend une quantité thérapeutiquement efficace ou une quantité cosmétiquement efficace de la culture cellulaire végétale telle que définie dans l'une quelconque des revendications 1-6, conjointement avec un excipient et/ou des porteurs pharmaceutiques ou cosmétiques acceptables quelconques.

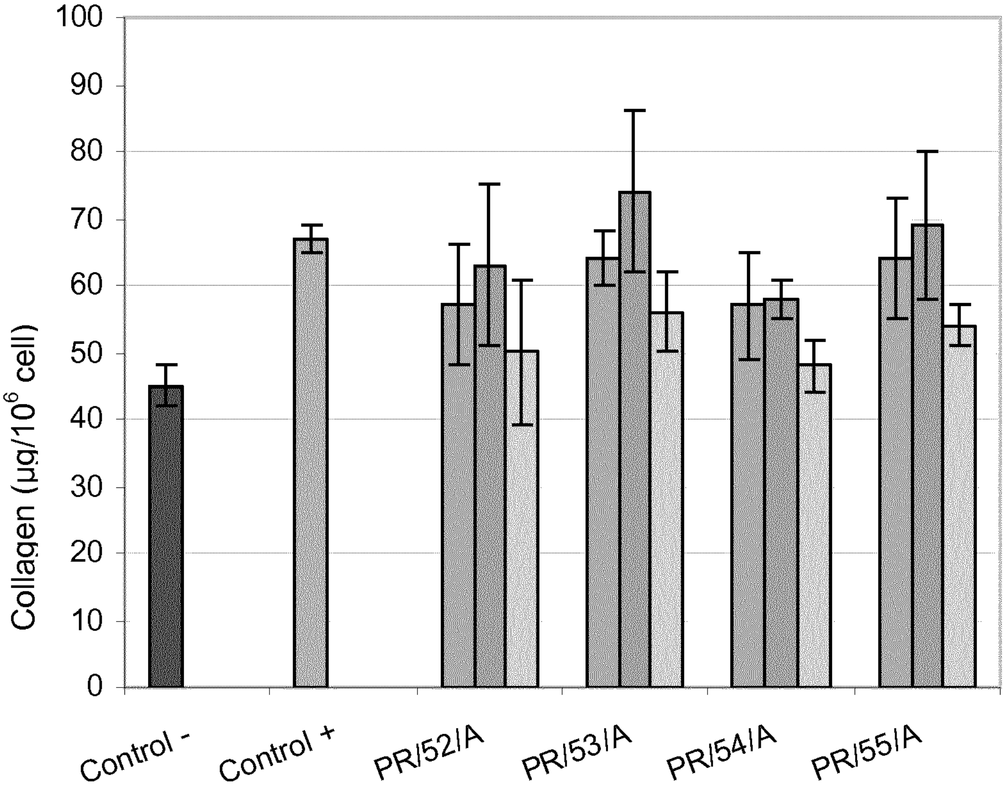

100
90
80
70
60
50
40
30
20
10
0
Collagen (µg/10^6 cell)
Control -
Control +
PR/52/A
PR/53/A
PR/54/A
PR/55/A


**FIG. 1**

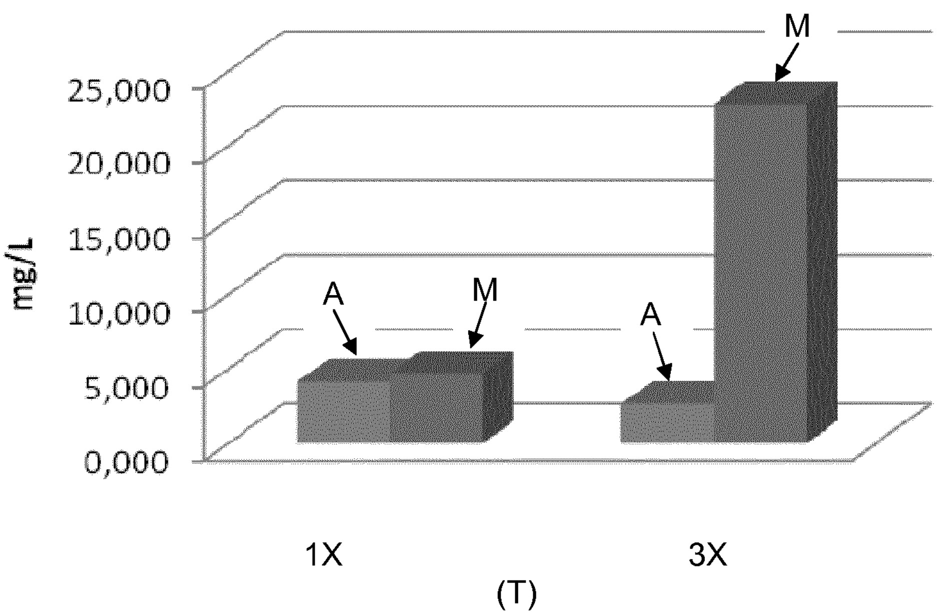


FIG. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 867447 A


### Non-patent literature cited in the description


- **MAQUART et al.** Stimulation of collagen synthesis in fibroblast cultures by a triterpene extracted from Centella asiatica. *Conn. Tissue Res,* 1990, vol. 24, 107-120
- **MULABAGAL et al.** Plant cell cultures-an alternative and efficient source for the production of biologically important secondary metabolites. *International Journal of Applied Science, Engineering and Technology,* 2004, vol. 2, 101-153
- **BONFILL et al.** Production of centellosides and phytosterols in cell suspension cultures of Centella asiatica. *Plant Cell Tiss Organ Cult,* 2011, (104), 61-67
- **MAQUART et al.** Stimulation of collagen synthesis in fibroblast cultures by a triterpene extracted from Centella asiatica. *Conn. Tissue Res-,* 1990, vol. 24, 107-120
- **MULABAGAL et al.** Plant cell cultures-an alternative and efficient source for the production of biologically important secondary metabolites. *International Journal of Applied Science, Engineering and Technology-,* 2004, vol. 2, 101-153
- **BONFILL et al.** Production of centellosides and phytosterols in cell suspension cultures of Centella asiatica. *Plant Cell Tiss Organ Cult-,* 2011, (104), 61-67